# EUROPEAN PATENT APPLICATION

(11) **EP 2 279 824 A1**
(43) Date of publication of application: **02.02.2011**
(21) Application number: 10007599.3
(22) Date of filing: 22.07.2010
(51) Int. Cl.: B23K 9/28, B23K 9/32, A61F 9/06

(54) **Electric arc welding mask and related electric arc welding method, with electronic radio receiving means**

(30) Priority: 30.07.2009 IT MI20091367
(71) Applicant: Tecnoelettra SPA, 23885 Calco (LC) (IT)
(72) Inventor: Brambilla, Fabrizio, 23807 Merate (LC) (IT)

(57) **Abstract**

Electric arc welding mask which comprises an electronic switching circuit and at least a liquid crystal glass able to switch through said electronic switching circuit from a rest condition in which shows a DIN shade inferior to 5 to a working condition in which shows a DIN shade greater than 9, the electric arc welding mask comprises electronic radio receiving means which are connected to the electronic switching circuit and are further able to receive a first switching radio command in order to activate the electronic switching circuit determining the switching of the at least a liquid crystal glass from the rest condition to the working condition before of an activation of an electric current able to determine the electric arc, avoiding consequently the need of the presence of the electric arc for activate the switching of the at least a liquid crystal glass and avoiding advantageously also a glare of brief time to the eyes of an operator.

Electric arc welding method through the use of at least an electric arc welding mask, comprising a phase of d) switch at least a liquid crystal glass and a phase of a) send to said at least an electric arc welding mask a first switching radio command if a start command of an electric arc welding has been activated.

## Description

The present invention refers to an electric arc welding mask and to a relative electric arc welding method.

Presently in the electric arc welding it is used a type of electric arc welding mask which is provided of at least a liquid crystal glass able to switch from a rest condition to a working condition in order to protect the eyes of an operator.

In the rest condition said at least a liquid crystal glass shows a DIN shade inferior to 5, while in the working condition shows a DIN shade greater than 9 in a way to filter the dangerous rays produced by an electric arc during an electric arc welding.

The passage from the rest condition to the working condition permit to filter the dangerous rays of the electric arc which is produced for determinate said electric arc welding.

The activation of the electric arc permit a following darkening of said welding mask.

In fact said electric arc welding mask is typically provided of a brightness sensor and of a switching circuit which is able to detect the luminous intensity produced by an electric arc during an arc welding and consequently to switch said liquid crystal glass from the rest condition to the working condition.

The switching from the rest condition to the working condition take place in a brief time included between 0,1 ms e 0,5 ms.

A first disadvantage is that during this brie range of time the eyes of an operator results however subject to a glare which repeated during the time however determines a damage to the eyes of the same operator.

Expensive masks are also provided with a electromagnetic field sensor which permits to detect the presence of the electric arc in such a way to activate through said switching circuit the switching of the electric arc welding mask from the rest condition to said working condition in order to protect the eyes of an operator.

Another disadvantage of this type of electric arc welding mask is that the presence of another electric arc, due to another operator in proximity while he perform another welding operation, and/or of other electronic instruments or devices in proximity of the arc welding mask, it often cause an interference between two or more welding mask determining frequently undesired darkening of the electric arc welding masks.

Besides in the case of a malfunctioning, if the arc welding mask do not switch from the rest condition to the working condition, it determines a glare of an operator for a much long time of the order of at least 2 or 3 seconds, until the electric arc is not interrupted.

This determines a damage to the eyes of the operator which is repeated each once that the mask break or that results malfunctioning.

Purpose of the present invention is that to realize an electric arc welding mask and a relative electric arc welding method which permit to avoid the glare to the eyes of an operator due to the electric arc when the electric arc welding mask is still not been switched from the rest condition to the working condition.

Another purpose is that to realize an electric arc welding mask and a relative electric arc welding method which permit to avoid the glare to the eyes of an operator in the case in which the arc welding mask, do not functioning correctly, do not switch from said rest condition to said working condition.

Another purpose is that to realize an electric arc welding mask and a relative electric arc welding method which avoid an undesired switching due to the interference with another electric arc or with other electric or electronic devices positioned near to the electric arc welding mask.

Further purpose is that to have an electric arc welding mask and a relative electric arc welding method that would be economically advantageous.

These purposes according to the present invention are reached realizing an electric arc welding mask and a relative electric arc welding method according to claims 1 and 11.

Further features of the invention are pointed out in the following claims.

The features and the advantages of an electric arc welding mask and of a relative electric arc welding method according to the present invention will appear more evident from the following illustrative and not limitative description.

According to the present invention it is provided an electric arc welding mask for the protection of the eyes of an operator which comprises an electronic switching circuit and at least a liquid crystal glass able to switch through said electronic switching circuit from a rest condition in which it shows a DIN shade inferior to 5 to a working condition in which it shows a DIN shade greater than 9 in such a way to filter the dangerous rays produced by an electric arc during an electric arc welding.

Said electric arc welding mask according to the present invention comprises electronic radio receiving means which are connected to said electronic switching circuit and are further able to receive a first switching radio command in order to activate said electronic switching circuit determining the switching of said at least a liquid crystal glass from said rest condition to said working condition before of an activation of an electric current able to determine said electric arc, avoiding consequently the need of the presence of said electric arc for activate said switching of said at least a liquid crystal glass and avoiding also a glare to the eyes of an operator.

This permit to avoid the need of the presence of said electric arc in order to perform said switching to said working condition, hence making possible the activation of the switching of said at least a liquid crystal glass before the beginning of said welding operation, hence avoiding advantageously the typical glares of the auto darkening welding mask of the prior art.

In fact it is possible to send said first radio switching command before to allow the passage of an electric current able to determine said electric arc.

Advantageously through said electronic radio receiving means it is possible to make the switching of said at least a liquid crystal glass independent by the presence of said electric arc, maintaining at the same time the same manageability and ease of use of said electric arc welding mask, because receiving by radio said first radio switching command it is possible to avoid the need of connection cables to the electric arc welding mask.

In other terms preferably said electric arc welding mask is radio controlled and permit to darken itself on the base of the activation of a welding start command before that it is allowed the passage of an electric current able to produce said electric arc.

In this way it is possible to make the darkening of said at least a liquid crystal glass, or the switching of the same from said rest condition to said working condition, independent from the presence of said electric arc.

Preferably said electric arc welding mask comprises electronic radio transmitting means which are able to send a third radio interrupt command to an electric arc welding torch or to an electric arc welding machine connected to an electric arc welding torch, in such a way to prevent the start of said electric arc welding in the case in which after the activation of said electronic switching circuit said at least a liquid crystal glass do not result correctly switched to said working condition.

Advantageously in this way it is possible to prevent the passage of said electric current necessary for produce said electric arc avoiding the start of said electric arc welding and consequently avoiding a glare to the eyes of an operator also if just a welding mask in proximity of a working zone do not have switched correctly said at least a liquid crystal glass from said rest condition to said working condition.

This increase notably the security avoiding the most frequent conditions of glare to the eyes of an operator, in a simple and effective manner.

When said at least a liquid crystal glass result switched to said working condition, preferably said electronic radio transmitting means are also able to send a second radio command to an electric arc welding torch or to an electric arc welding machine connected to an electric arc welding torch, in such a way to start said welding operation in the case it is known the total number of electric arc welding masks in that predetermined working zone.

Preferably said electric arc welding mask comprises modulation/demodulation means of signals in frequency which are connected or preferably integrated with said electronic radio receiving means in such a way to modulate and demodulate said first switching radio command in order to avoid an undesired switching of another electric arc welding mask in proximity of said electric arc welding mask.

In particular said first radio switching command and preferably also said third radio interrupt command are frequency modulated, in such a way to have different radio commands of activation of the darkening or of interruption.

Advantageously this permit also a synchronization of more electric arc welding masks with a same electric arc torch or with a same electric arc welding machine, other than avoid possible undesired switching between two or more electric arc welding masks.

According to a preferred form of embodiment said electronic radio receiving means comprise electronic demodulation means of signals in frequency in order to demodulate said first radio switching command.

In particular said electronic radio transmitting means comprises electronic modulation means of signals in frequency in order to avoid an undesired switching of at least a liquid crystal glass of an electric arc welding mask in proximity of others electric arc welding masks.

In particular said electric arc welding mask comprises selection means of a radio modulation frequency, in such a way to select a radio modulation frequency in order to avoid an undesired switching or in order to obtain at the same time a switching of two or more electric arc welding masks when it is activated a welding start command of a same electric arc welding torch or of a same electric arc welding machine.

Advantageously this permits to weld with different operating conditions maintaining an high protection level of the eyes of one or more operators near to one or more electric arc.

According to another aspect of the present invention it is provided an electric arc welding torch comprising electronic radio transmitting means which are able to send a first switching radio command to at least an electric arc welding mask provided of at least a liquid crystal glass in order to switch said at least a liquid crystal glass from a rest condition in which shows a DIN shade inferior to 5 to a working condition in which shows a DIN shade greater than 9 before of an activation of an electric current able to determine an electric arc, avoiding therefore the need of the presence of said electric arc for activate said switching from said rest condition to said working condition and avoiding therefore a glare to the eyes of an operator.

Advantageously with said electric arc welding torch, and with at least an electric arc welding mask of the type previously described, it is possible to avoid the glares of the prior art besides permitting to one or more operators to use a standard electric arc welding machine without replace or modify the same.

Preferably said electric arc welding torch comprises electronic radio receiving means which are able to receive a third radio interrupt command coming from said at least an electric arc welding mask in order to prevent the start of said electric arc welding in the case in which said at least an electric arc welding mask after the receipt of said first switching radio command do not have switched said at least a liquid crystal glass to said working condition.

Advantageously in this way it is possible to prevent the passage of said electric current that it is necessary for produce said electric arc therefore avoiding the start of said electric arc welding, consequently avoiding a possible glare to the eyes of an operator, also if only one electric arc welding mask in proximity of a working zone do not result switched from said rest condition to said working condition.

In particular said electric arc welding torch comprises modulation/demodulation means of signal in frequency which are connected or preferably integrated with said electronic radio transmitting means in such a way to modulate in frequency said first switching radio command in order to avoid an undesired switching of another electric arc welding mask in proximity of said electric arc welding mask.

According to another aspect of the present invention it is provided an electric arc welding machine comprising an electric generator able to supply an electric current in order to determine an electric arc and besides comprising electronic radio transmitting means which are able to send a first switching radio command to at least an electric arc welding mask provided of at least a liquid crystal glass in order to switch said at least a liquid crystal glass from a rest condition in which shows a DIN shade inferior to 5 to a working condition in which shows a DIN shade greater than 9 before of an activation of an electric current able to determine said electric arc, avoiding therefore the need of the presence of said electric arc for activate said switching from said rest condition to said working condition and avoiding therefore a glare to the eyes of an operator.

Advantageously with said electric arc welding machine, and with at least an electric arc welding mask of the type previously described, it is possible to avoid the glares of the prior art besides permitting to one or more operators to use one or more standard electric arc welding torches without have to replace or modify the same.

Preferably said electric arc welding machine comprises electronic radio receiving means which are able to receive a third radio interrupt command coming from said at least an electric arc welding mask in order to prevent the start of said electric arc welding in the case in which said at least an electric arc welding mask after the receipt of said first switching radio command do not have switched said at least a liquid crystal glass to said working condition.

Advantageously in this way it is possible to prevent the passage of said electric current that it is necessary for produce said electric arc therefore avoiding the start of said electric arc welding, consequently avoiding a possible glare to the eyes of an operator also if only one electric arc welding mask in proximity of a working zone do not result switched from said rest condition to said working condition.

In particular said electric arc welding machine comprises modulation/demodulation means of signal in frequency which are connected or preferably integrated with said electronic radio transmitting means in such a way to modulate in frequency said first switching radio command in order to avoid an undesired switching of another electric arc welding mask in proximity of said electric arc welding mask.

According to another aspect of the present invention it is provided an electric arc welding equipment comprising at least an electric arc welding mask according to any one form previously described, and besides comprising at least an electric arc welding torch according any one form previously described and/or an electric arc welding machine according any one form previously described.

According to another aspect of the present invention it is provided an electric arc welding method through the use of at least an electric arc welding mask provided of at least a liquid crystal glass able to switch from a rest condition in which shows a DIN shade inferior to 5 to a working condition in which shows a DIN shade greater than 9 in such a way to filter the dangerous rays produced by an electric arc during an electric arc welding.

Said electric arc welding method comprises a phase of d) switch said at least a liquid crystal glass from said rest condition to said working condition.

According to the present invention said electric arc welding method comprises the following phases:
a) send to said at least an electric arc welding mask a first switching radio command if a start command of said electric arc welding has been activated from an electric arc welding torch or from an electric arc welding machine;
b) receive said first switching radio command through said at least an electric arc welding mask;
c) if said first switching radio command has been received through said phase b) then perform said phase d) before to allow the passage of an electric current able to determine said electric arc, so avoiding to glare also just for a brief time the eyes of an operator.

In this way it is possible to switch said at least a liquid crystal glass advantageously avoiding the need of the existence of said electric arc for perform said d) phase.

Advantageously in this way it is possible to darken said at least a liquid crystal glass through the switching of the same from said rest condition to said working condition before that it was been activated an electric current able to determine said electric arc, in such a way to protect the eyes of an operator before to activate said electric arc, avoiding advantageously also the typical little glare of the prior art.

Besides preferably said method comprises a phase of :
h) if it has not received at least a third radio interrupt command through said electric arc welding torch or through said electric arc welding machine connected to an electric arc welding torch, then allow the passage of an electric current able to determine said electric arc.

Advantageously in this way it is possible to be sure to activate said electric arc only when said at least a liquid crystal glass is correctly switched to said working condition, in other words only when the eyes of an operator results protected.

Said a), b), c), d) and h) phases are performed in this sequence in such a way to switch said at least a liquid crystal glass before to allow the passage of an electric current able to determine said electric arc, in a way to avoid the typical glares of the prior art.

Besides preferably said method comprises the sequent phase:
g) receive said at least a third radio interrupt command , in particular coming from said at least an electric arc welding mask, through an electric arc welding torch or through an electric arc welding machine connected to an electric arc welding torch which are preferably positioned in proximity of said at least an electric arc welding mask.

Preferably said g) phase is performer for at least 0,1 s from the sending of said first switching radio command, and in particular for at least 0,5 s.

Besides preferably said method comprises the sequent phase:
e) if said at least a liquid crystal glass after said phase d) do not results switched to said working condition then f) send a third radio interrupt command to an electric arc welding torch or to an electric arc welding machine connected to an electric arc welding torch in order to prevent the start of said electric arc welding.

Advantageously in this way it is possible to prevent that the eyes of an operator will be glared in the case that also only one electric arc welding mask do not result switched to said working condition.

This advantageously permit to solve the problem of a glare in the case of bad working of an electric arc welding mask.

In this way it is possible to permit the passage of said electric current for produce said electric arc only if all the electric arc welding masks results switched to the working condition.

Preferably said method comprises besides the following phase:
i) modulate/demodulate in frequency said first switching radio command and preferably also said third radio interrupt command in order to avoid an undesired switching of another electric arc welding mask in proximity of said at least an electric arc welding mask.

Preferably said first radio switching command and said third radio interrupt command are therefore modulated in frequency in order to avoid noises from electronic devices possibly in proximity to said electric arc welding mask.

Advantageously this permit also to avoid that two electric arc welding masks positioned in proximity of two electric arcs could be wrongly switched, because in this way it is possible to select a different frequency of modulation in order to operate in an independent way also having two electric arcs proximal one to the other.

Preferably said method comprises a phase of m) select a communication frequency, in such a way to avoid an undesired switching of another electric arc welding mask in proximity of said at least an electric arc welding mask.

Advantageously this permit at two or more operators to perform two different electric arc welding in two near positions simply selecting two different radio communication frequencies, in such a way to avoid an undesired switching when another operator start an electric arc welding.

Preferably said method comprises a phase of p) activate a start welding command.

Preferably said method comprises a phase of q) measure said electric current obtaining an instant electric welding current and a phase of r) adjust a DIN shade of said working condition in a proportional way to said instant electric welding current.

In particular said phase r) comprises a phase of s) send to said at least an electric arc welding machine a fourth radio adjust command preferably modulated in frequency in such a way to determine an adjust of said DIN shade of said working condition in automatic way depending by said instant electric welding current.

Preferably said method comprises a phase of t) display on said electric arc welding mask an alarm signal, and in particular to display on at least a liquid crystal glass an alarm signal, in such a way to avoid dangerous situations for an operator.

Preferably said method comprises the sequent phases:
u) detect at least a functioning parameter of an electric arc welding machine;
v) display on said at least a liquid crystal glass said at least a functioning parameter of said electric arc welding machine in order to check the working conditions of said electric arc welding machine in such a way to highlight a problem or a malfunctioning.

In this way it has been seen that an electric arc welding mask and the relative electric arc welding method to the present invention achieves the previously mentioned goals.

The electric arc welding mask and the relative electric arc welding method thus conceived can undergo to numerous modifications and variations, all included in the same inventive concept.

Furthermore, in practice the materials used, as well as their dimensions and the components, can vary according to the technical needs.

## Claims

1. Electric arc welding mask for the protection of the eyes of an operator which comprises an electronic switching circuit and at least a liquid crystal glass able to switch through said electronic switching circuit from a rest condition in which shows a DIN shade inferior to 5 to a working condition in which shows a DIN shade greater than 9 in such a way to filter the dangerous rays produced by an electric arc during an electric arc welding, said electric arc welding mask **characterized by** comprising electronic radio receiving means which are connected to said electronic switching circuit and are further able to receive a first switching radio command in order to activate said electronic switching circuit determining the switching of said at least a liquid crystal glass from said rest condition to said working condition before of an activation of an electric current able to determine said electric arc, avoiding consequently the need of the presence of said electric arc for activate said switching of said at least a liquid crystal glass and avoiding also a glare to the eyes of an operator.

2. Electric arc welding mask according to claim 1, **characterized by** comprising electronic radio transmitting means which are able to send a third radio interrupt command to an electric arc welding torch or to an electric arc welding machine connected to an electric arc welding torch, in such a way to prevent the start of said electric arc welding in the case in which after the activation of said electronic switching circuit said at least a liquid crystal glass do not result correctly switched to said working condition.

3. Electric arc welding mask according to claim 1 or 2, **characterized by** comprising modulation/demodulation means of signals in frequency which are connected or preferably integrated with said electronic radio receiving means in such a way to demodulate said first switching radio command in order to avoid an undesired switching of another electric arc welding mask in proximity of said electric arc welding mask.

4. Electric arc welding torch **characterized by** comprising electronic radio transmitting means which are able to send a first switching radio command to at least an electric arc welding mask provided of at least a liquid crystal glass in order to switch said at least a liquid crystal glass from a rest condition in which shows a DIN shade inferior to 5 to a working condition in which shows a DIN shade greater than 9 before of an activation of an electric current able to determine an electric arc, avoiding therefore the need of the presence of said electric arc for activate said switching from said rest condition to said working condition and avoiding therefore a glare to the eyes of an operator.

5. Electric arc welding torch according to claim 4, **characterized by** comprising electronic radio receiving means which are able to receive a third radio interrupt command coming from said at least an electric arc welding mask in order to prevent the start of said electric arc welding in the case in which said at least an electric arc welding mask after the reception of said first switching radio command do not have switched said at least a liquid crystal glass to said working condition.

6. Electric arc welding torch according to claim 4 or 5, **characterized by** comprising modulation/demodulation means of signal in frequency which are connected or preferably integrated with said electronic radio transmitting means in such a way to modulate in frequency said first switching radio command in order to avoid an undesired switching of another electric arc welding mask in proximity of said electric arc welding mask.

7. Electric arc welding machine comprising an electric generator able to supply an electric current in order to determine an electric arc, **characterized by** comprising electronic radio transmitting means which are able to send a first switching radio command to at least an electric arc welding mask provided of at least a liquid crystal glass in order to switch said at least a liquid crystal glass from a rest condition in which shows a DIN shade inferior to 5 to a working condition in which shows a DIN shade greater than 9 before of an activation of an electric current able to determine said electric arc, avoiding therefore the need of the presence of said electric arc for activate said switching from said rest condition to said working condition and avoiding therefore a glare to the eyes of an operator.

8. Electric arc welding machine according to claim 7, **characterized by** comprising electronic radio receiving means which are able to receive a third radio interrupt command coming from said at least an electric arc welding mask in order to prevent the start of said electric arc welding in the case in which said at least an electric arc welding mask after the reception of said first switching radio command do not have switched said at least a liquid crystal glass to said working condition.

9. Electric arc welding machine according to claim 7 or 8, **characterized by** comprising modulation/demodulation means of signal in frequency which are connected or preferably integrated with said electronic radio transmitting means in such a way to modulate in frequency said first switching radio command in order to avoid an undesired switching of another electric arc welding mask in proximity of said electric arc welding mask.

10. Electric arc welding equipment comprising at least an electric arc welding mask according to any one of the claims from 1 to 3 and besides comprising at least an electric arc welding torch according to any one of the claims from 4 to 6 and/or at least an electric arc welding machine according to any one of the claims from 7 to 9.

11. Electric arc welding method through the use of at least an electric arc welding mask provided of at least a liquid crystal glass able to switch from a rest condition in which shows a DIN shade inferior to 5 to a working condition in which shows a DIN shade greater than 9 in such a way to filter the dangerous rays produced by an electric arc during an electric arc welding, said electric arc welding method comprises a phase of d) switch said at least a liquid crystal glass from said rest condition to said working condition, said electric arc welding method **characterized by** comprising the following phases:
a) send to said at least an electric arc welding mask a first switching radio command if a start command of said electric arc welding has been activated from an electric arc welding torch or from an electric arc welding machine;
b) receive said first switching radio command through said at least an electric arc welding mask;
c) if said first switching radio command has been received through said phase b) then perform said phase d) before to allow the passage of an electric current able to determine said electric arc, avoiding therefore to glare also just for a brief time the eyes of an operator.

12. Electric arc welding method according to claim 11, **characterized by** comprising a phase of :
h) if it has not received at least a third radio interrupt command through said electric arc welding torch or through said electric arc welding machine connected to an electric arc welding torch, then allow the passage of an electric current able to determine said electric arc.

13. Electric arc welding method according to claim 11 or 12, **characterized by** comprising a phase of :
g) receive said at least a third radio interrupt command , in particular coming from said at least an electric arc welding mask, through an electric arc welding torch or through an electric arc welding machine connected to an electric arc welding torch which are preferably positioned in proximity of said at least an electric arc welding mask.

14. Electric arc welding method according to any one of the claims from 11 to 13, **characterized by** comprising a phase of :
e) if said at least a liquid crystal glass after said phase d) do not results switched to said working condition then f) send a third radio interrupt command to an electric arc welding torch or to an electric arc welding machine connected to an electric arc welding torch in order to prevent the start of said electric arc welding.

15. Electric arc welding method according to any one of the claims from 11 to 14, **characterized by** comprising a phase of :
i) modulate/demodulate in frequency said first switching radio command and preferably also said third radio interrupt command in order to avoid an undesired switching of another electric arc welding mask in proximity of said at least an electric arc welding mask.

16. Electric arc welding method according to claim 15, **characterized by** comprising a phase of :
m) select a communication frequency, in such a way to avoid an undesired switching of another electric arc welding mask in proximity of said at least an electric arc welding mask.

17. Electric arc welding method according to any one of the claims from 11 to 16, **characterized by** comprising a phase of q) measure said electric current obtaining an instant electric welding current and a phase of r) adjust a DIN shade of said working condition in a proportional way to said instant electric welding current.

18. Electric arc welding method according to claim 17, **characterized in that** said phase r) comprises a phase of s) send to said at least an electric arc welding machine a fourth radio adjust command preferably modulated in frequency in such a way to determine an adjust of said DIN shade of said working condition in automatic way depending by said instant electric welding current.

19. Electric arc welding method according to any one of the claims from 11 to 18, **characterized by** comprising the sequent phases:
u) detect at least a functioning parameter of an electric arc welding machine;
v) display on said at least a liquid crystal glass said at least a functioning parameter of said electric arc welding machine in order to check the working conditions of said electric arc welding machine in such a way to highlight a problem or a malfunctioning.
